# EUROPÄISCHE PATENTSCHRIFT

(11) **EP 3 171 910 B1**
(45) Veröffentlichungstag und Bekanntmachung des Hinweises auf die Patenterteilung: **28.08.2019**
(21) Anmeldenummer: 15739520.3
(22) Anmeldetag: 20.07.2015
(51) Int. Cl.: A61M 1/16, A61M 1/34, A61M 1/36

(54) **VORRICHTUNG ZUR EXTRAKORPORALEN ENTFERNUNG PROTEINGEBUNDENER TOXINE**
DEVICE FOR EXTRACORPOREAL REMOVAL OF PROTEIN-BOUND TOXINS
PROCÉDÉ D'ÉLIMINATION EXTRACORPORELLE DE TOXINES LIÉES AUX PROTÉINES

(30) Priorität: 23.07.2014 DE 102014010907
(43) Veröffentlichungstag der Anmeldung: 31.05.2017
(73) Patentinhaber: Fresenius Medical Care Deutschland GmbH, 61352 Bad Homburg (DE)
(72) Erfinder: FABIG, Anselm, 15738 Zeuthen (DE); TSCHULENA, Ulrich, 60439 Frankfurt am Main (DE); STEPPAN, Sonja, 63263 Neu-Isenburg (DE)
(74) Vertreter: Herrmann, Uwe
(86) Internationale Anmeldenummer: PCT/EP2015/001499
(87) Internationale Veröffentlichungsnummer: WO 2016/012093

(56) Entgegenhaltungen:
- WO-A1-2014/095072
- US-A- 5 188 738

## Beschreibung

Die vorliegende Erfindung betrifft eine Vorrichtung zur extrakorporalen Entfernung proteingebundener Toxine aus dem Blut umfassend wenigstens eine Blutreinigungsvorrichtung, insbesondere wenigstens einen Dialysator, Hämofilter oder Adsorber, sowie wenigstens ein Mittel zur Erzeugung eines Feldes in der Blutreinigungsvorrichtung und/oder in einem mit der Blutreinigungsvorrichtung in Strömungsverbindung stehenden Element, bei dem es sich vorzugsweise um wenigstens einen mit der Blutreinigungsvorrichtung in Verbindung stehenden Leitungsabschnitt handelt.

Bei gesunden Personen werden verschiedene im Blut gelöste Substanzen, wie beispielsweise urämische Toxine über die Niere mit dem Harn ausgeschieden. Liegt ein Nierenversagen vor bzw. können die Nieren diese Reinigungsfunktion nicht mehr oder nur noch eingeschränkt erfüllen, müssen die fraglichen Stoffe mittels eines Blutreinigungsverfahrens, wie beispielsweise mittels der Hämodialyse oder Peritonealdialyse aus dem Blut entfernt werden.

Wasserlösliche Toxine, wie beispielsweise Harnstoff können mit den bekannten Blutreinigungsverfahren ohne weiteres aus dem Blut entfernt werden. Hingegen ist die Entfernung von schlecht wasserlöslichen hydrophoben urämischen Toxinen mittels der bekannten Blutreinigungsverfahren aufgrund der Proteinbindung dieser Toxine erschwert.

Als Bindungspartner für urämische Toxine kommen Plasmaproteine in Betracht, insbesondere Albumin, das aufgrund seiner Größe von üblichen Dialysemembranen zurückgehalten wird. Dies gilt entsprechend für urämische Toxine, die mit Albumin eine Bindung eingehen. Zwar besteht ein Gleichgewicht zwischen den freien urämischen Toxinen und den proteingebundenen urämischen Toxinen, das dazu führt, dass bei hinreichend langer Blutbehandlung ein wesentlicher Teil der urämischen Toxine mittels der Blutreinigungsvorrichtung entfernt wird, jedoch steht eine solche lange Zeitdauer für die Behandlung üblicherweise nicht zur Verfügung.

Aus der WO 2014/095072 A1 ist eine Vorrichtung bekannt, die Mittel zur Erzeugung eines elektromagnetischen Feldes umfasst, das auf das Blut einwirkt. Hintergrund für diese Vorgehensweise ist es, dass durch die Wirkung des Feldes die Stärke der Bindungen bzw. die Wechselwirkungen zwischen den urämischen Toxinen und den Proteinen herabgesetzt wird, wodurch der Anteil der proteingebunden urämischen Toxine deutlich reduziert werden kann. Demzufolge lässt sich bei der Blutreinigung eine verbesserte Abtrennung der urämischen Toxine aus dem Blut eines Patienten erzielen. Zur Erzeugung des Feldes werden beispielsweise Kondensatoren eingesetzt.

Der vorliegenden Erfindung liegt die Aufgabe zugrunde, eine Vorrichtung der eingangs genannten Art dahingehend weiterzubilden, dass die Effizienz der Abtrennung der urämischen Toxine von den Plasmaproteinen gegenüber der bekannten Vorrichtung verbessert wird.

Diese Aufgabe wird durch eine Vorrichtung mit den Merkmalen des Anspruchs 1 gelöst.

Danach ist vorgesehen, dass das Mittel wenigstens zwei Streifenleiter umfasst, die derart an der Blutreinigungsvorrichtung bzw. an dem Element angeordnet sind, dass sich das Feld, insbesondere ein elektromagnetisches Feld, innerhalb der Blutreinigungsvorrichtung und/oder innerhalb des Elementes ausbreitet. Das Feld breitet sich somit vorwiegend innerhalb der Blutreinigungsvorrichtung bzw. innerhalb des Elementes aus, was auch den Fall mit einschließt, dass sich das Feld ausschließlich innerhalb der Blutreinigungsvorrichtung bzw. innerhalb des Elementes ausbreitet.

Bei dem genannten Element kann es sich beispielsweise um eine Leitung oder einen Leitungsabschnitt oder eine sonstige Komponente handeln, durch den/die das Blut oder ein Teil von diesem zu der Blutreinigungsvorrichtung strömt.

Die Anordnung der Streifenleiter auf der Blutreinigungsvorrichtung bzw. auf dem genannten Element hat den Vorteil, dass das Feld vollständig oder weitgehend durch die Blutreinigungsvorrichtung, wie beispielsweise durch einen Dialysator, oder durch das genannte Element hindurch verläuft und somit besonders wirksam die Auftrennung der bestehenden Bindungen zwischen Plasmaproteinen, insbesondere Albumin und urämischen Toxinen im Blut bewirkt.

Ein weiterer Vorteil der Anordnung liegt darin, dass sich das Feld vorzugsweise überwiegend innerhalb der Blutreinigungsvorrichtung ausbreitet. Da das εᵣ von Luft ungefähr gleich 1 und damit sehr viel kleiner als das εᵣ von Wasser bzw. Blut ist, wird das Feld nur zu einem geringeren Teil in die umgebende Luft abgestrahlt. Dies hat den Vorteil, dass an die Abschirmung der Blutreinigungsvorrichtung geringere Anforderungen gestellt werden und u.U. auf eine allseitige Abschirmung gänzlich verzichtet werden kann.

Die wenigstens zwei Streifenleiter sind auf der Außenseite der Blutreinigungsvorrichtung oder des Elementes und bevorzugt unmittelbar oder mittelbar auf der Außenseite des Gehäuses der Blutreinigungsvorrichtung oder des Elementes angeordnet.

In einer bevorzugten Ausgestaltung der Erfindung ist vorgesehen, dass die Streifenleiter derart mit der Blutreinigungsvorrichtung oder mit dem Element in Verbindung stehen, dass sich zwischen den Streifenleitern und der Blutreinigungsvorrichtung bzw. dem Element keine Luft befindet. Bevorzugt ist somit eine luftfreie Anbindung der Streifenleiter an die Blutreinigungsvorrichtung bzw. an das Element.

Die Streifenleiter sind beispielsweise derart angeordnet, dass sie auf zwei gegenüberliegenden Seiten der Blutreinigung bzw. des Elementes und vorzugsweise symmetrisch zu einer Längsebene der Blutreinigungsvorrichtung oder des Elementes verlaufen.

Die Mittel können genau zwei oder auch mehr als zwei Streifenleiter umfassen.

Die Streifenleiter können beispielsweise mit der Blutreinigungsvorrichtung oder mit dem Element verklebt sein oder an der Blutreinigungsvorrichtung oder an dem Element z.B. durch eine oder mehrere Klammern etc. mechanisch befestigt sein.

Um eine gute, luftfreie Anbindung der Streifenleiter an die Blutreinigungsvorrichtung oder an das Element zu erreichen, kann vorgesehen sein, dass sich zwischen den Streifenleitern und der Blutreinigungsvorrichtung bzw. dem Element ein wasserhaltiges Gel befindet. In Betracht kommt beispielsweise in Gel, wie es bei Ultraschallanwendungen zum Einsatz kommt.

Die Mittel zur Erzeugung eines Feldes können derart ausgebildet sein, dass ein elektromagnetisches Feld mit einer Frequenz im Bereich von 1 MHz bis 1 GHz und vorzugsweise mit einer Frequenz im Bereich von 50 MHz bis 200 MHz und besonders bevorzugt mit einer Frequenz im Bereich von 100 MHz bis 120 MHz erzeugt wird.

Als besonders geeignet erweist sich eine Frequenz im Bereich 110 MHz bis 111 MHz.

Weiterhin kann vorgesehen sein, dass das Mittel zur Erzeugung eines Feldes derart ausgebildet ist, dass das Feld eine Feldstärke im Bereich von bis zu 4000 V/m, vorzugsweise im Bereich von 2000 V/m bis 4000 V/m und besonders bevorzugt im Bereich von 3000 V/m bis 3200 V/m aufweist.

Bei dem durch die Streifenleiter erzeugten Feld kann es sich um ein hochfrequentes elektromagnetisches Feld mit überlagertem elektrischem Gleichfeld handeln.

Die Blutreinigungsvorrichtung oder das Element kann wenigstens bereichsweise eine gekrümmte Oberfläche aufweisen. So ist es beispielsweise denkbar, dass es sich bei der Blutreinigungsvorrichtung um einen Dialysator mit einem zylindrischen Gehäuse handelt und/oder bei dem Element um eine Komponente, wie z.B. ein Schlauch, ein Rohr etc. mit einer gekrümmten Wandung. Die Streifenleiter können ebenfalls gekrümmt ausgebildet sein, so dass sie der Kontur der Oberfläche der Blutreinigungsvorrichtung oder des Elementes folgen.

Wie oben ausgeführt, ist es denkbar, dass die Streifenleiter auf die Außenseite des Gehäuses der Blutreinigungsvorrichtung oder des Elementes aufgeklebt oder dort anderweitig fixiert sind und somit im Falle einer gekrümmten Oberfläche ebenfalls eine Krümmung aufweisen.

Von der Erfindung ist selbstverständlich auch der Fall umfasst, dass die Blutreinigungsvorrichtung oder das Element eine ebene Oberfläche aufweist und die Streifenleiter somit ebenfalls eben ausgebildet sind.

Denkbar ist es, dass die Streifenleiter teilweise oder vollständig als Folie oder als plattenförmiges Element oder als die Blutreinigungsvorrichtung bzw. das Element teilweise oder vollständig umgreifende Elemente bzw. Halter ausgebildet sind.

Die Streifenleiter bestehen vorzugsweise teilweise oder vollständig aus Metall. Vorzugsweise ist der Einsatz von Kupfer oder Aluminium vorgesehen.

Denkbar ist es, dass sich die Streifenleiter über die gesamte Länge der Blutreinigungsvorrichtung bzw. des Elementes oder einen Teil der gesamten Länge, vorzugsweise über mehr als die Hälfte der Länge der Blutreinigungsvorrichtung bzw. des Elementes erstrecken. Weist die Blutreinigungsvorrichtung bzw. das genannte Element beispielsweise eine langgestreckte Form auf, kann vorgesehen sein, dass sich die Streifenleiter über die gesamte Länge oder zumindest über einen überwiegenden Teil der Länge der Blutreinigungsvorrichtung bzw. des Elementes erstrecken.

Vorzugsweise ist vorgesehen, dass die Streifenleiter derart dimensioniert sind, dass das von diesen erzeugte Feld den gesamten Raum, in dem sich das Blut befindet, oder einen überwiegenden Teil dieses Raums durchdringt.

Vorzugsweise weist die Vorrichtung wenigstens einen extrakorporalen Kreislauf auf, in dem sich die Blutreinigungsvorrichtung und/oder das genannte Element befinden.

Wie oben ausgeführt, kann es sich bei der Blutreinigungsvorrichtung beispielsweise um einen Dialysator oder einen Hämofilter handeln, der in einer Hämofiltrationsbehandlung oder bei der Hämodiafiltration zum Einsatz kommt. Alternativ oder zusätzlich kann es sich bei der Blutreinigungsvorrichtung auch um einen Adsorber oder dergleichen handeln, mit dem bestimmte Stoffe aus dem Blut durch Adsorption entfernt werden.

Der Adsorber kann ein Anionentauscher oder ein hydrophober Adsorber sein, beispielsweise Aktivkohle, die vorzugsweise eine hämokompatible Beschichtung aufweist. Er hält Toxine zurück und verhindert auf diese Weise, dass diese dem Patienten wieder zugeführt werden.

Weiterhin kann vorgesehen sein, dass der extrakorporale Kreislauf wenigstens einen Filter umfasst, der eine Permeatseite und eine Retentatseite umfasst, wobei die Blutreinigungsvorrichtung mit der Permeatseite in Verbindung steht. Auch ein Zellseparator kann eingesetzt werden, der das Blut in Blutplasma und in einen zellulären Bestandteil trennt, wobei der Zellseparator derart verschaltet ist, dass das Blutplasma, nicht jedoch die zellulären Bestandteile des Blutes in die Blutreinigungsvorrichtung gelangen.

Die erfindungsgemäße Vorrichtung kann zur Verwendung in der Behandlung des akuten oder chronischen Nierenversagens dienen.

Die erfindungsgemäße Vorrichtung kann auch zur Verwendung in der Behandlung des akuten oder chronischen Leberversagens dienen.

Die erfindungsgemäße Vorrichtung kann auch zur Verwendung in der Behandlung von Patienten mit einer Sepsis dienen oder zur Verwendung in der Behandlung von Patienten mit einer Vergiftung in einer Vorrichtung zur Entgiftung der Patienten.

Weitere Einzelheiten und Vorteile der Erfindung werden anhand eines in der Zeichnung dargestellten Ausführungsbeispiels näher erläutert.

Es zeigen:
- Figur 1:: eine schematische Querschnittansicht durch einen Dialysator mit auf gegenüberliegenden Seiten angeordneten Streifenleitern,
- Figur 2:: eine Detailansicht eines Abschnittes des Dialysators mit auf dessen Außenseite angeordnetem Streifenleiter,
- Figur 3:: eine schematische Ansicht einer Vorrichtung gemäß der Erfindung mit Plasmafilter und Dialysator und
- Figur 4:: eine schematische Ansicht einer Vorrichtung gemäß der Erfindung mit Plasmafilter und Dialysator sowie Adsorber.

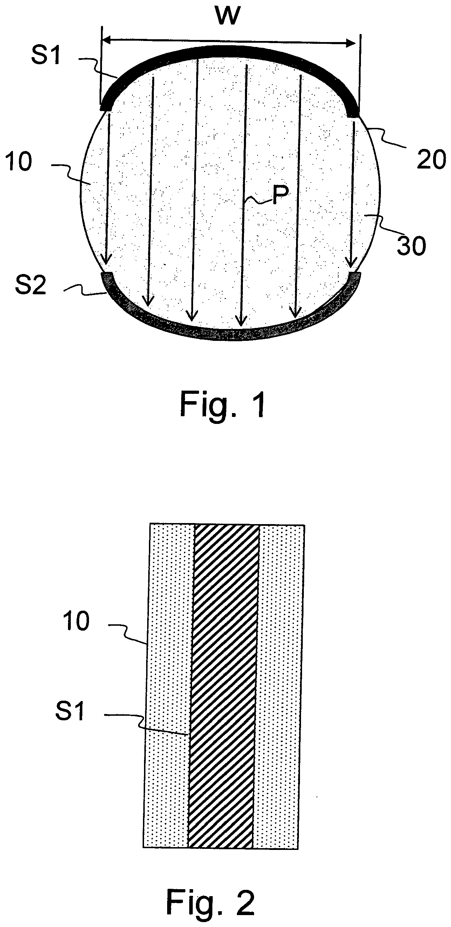

Figur 1 zeigt in einer schematischen Querschnittsansicht einen Dialysator 10, der ein zylindrisches Gehäuse 20 aufweist, in dessen Innenraum 30 sich ein nicht dargestelltes Faserbündel befindet. Bei der Behandlung werden die Fasern des Faserbündels von Blut durchströmt, der das Faserbündel umgebende Bereich wird von der Dialyselösung umströmt.

Die Blutreinigung erfolgt durch Diffusion über die als Membranen ausgeführten Wandungen der Fasern.

Von der Erfindung ist ebenso jede andere Blutreinigungsvorrichtung umfasst, wie beispielsweise ein Hämofilter, bei dem durch einen Druckgradienten ein Stoffübergang über die Fasermembranen erfolgt, oder auch ein Hämodiafilter, bei dem die Blutreinigung sowohl aufgrund eines Konzentrationsgradienten als auch aufgrund eines Druckgradienten über die Fasermembranen erfolgt. Auch andere Blutreinigungsvorrichtungen, wie beispielsweise ein Adsorber sind von der Erfindung mit umfasst.

Wie dies weiter aus Figur 1 hervorgeht, befinden sich relativ zu einer senkrecht zur Papierebene verlaufenden Mittelebene durch den Dialysator 10 symmetrisch angeordnete Streifenleiter S1 und S2, die entsprechend der Krümmung des Dialysatorgehäuses gebogen sind, wie dies aus Figur 1 hervorgeht. Die Streifenleiter werden derart mit Strom/Spannung beaufschlagt, dass sich zwischen diesen ein Feld ausbildet, das sich innerhalb des Dialysators befindet.

Die Pfeile P in Figur 1 kennzeichnen ein zwischen Streifenleitern verlaufendes Feld, bei dem sich vorzugsweise um ein elektromagnetisches Feld und besonders bevorzugt um ein hochfrequentes elektromagnetisches Feld handelt.

Die Breite W der Streifenleiter S1 und S2 in Figur 1 ist so gewählt, dass sich ein Wellenwiderstand von 50 Ω ergibt.

Geht man von einem Durchmesser des Dialysators 10 von 32 mm aus, lässt sich bei Einsatz eines 200 W Verstärkers an 50 Ω und bei einer Frequenz in der Spanne von 110 - 111 MHz ein Feld von 3125 V/m erzielen.

In der in Figur 1 dargestellten Anordnung sind die Streifenleiter luftdicht, d.h. ohne ein Luftpolster oder Lufteinschlüsse unmittelbar oder mittelbar mit der Außenseite des Dialysatorgehäuses verbunden. Die Verbindung kann durch Kleben, durch mechanische Mittel etc. erfolgen.

Aus Figur 1 ist weiter ersichtlich, dass sich die Streifenleiter auf gegenüberliegenden Seiten des Dialysators erstrecken. Sie umgreifen jeweils einen Teilbereich des Dialysators, weitere Teilbereiche des Dialysatorumfangs sind nicht mit Streifenleitern versehen, wie dies aus Figur 1 ersichtlich ist.

Aus Figur 2 ist eine Ansicht eines Teilbereiches des Dialysators 10 ersichtlich. Auf die Außenoberfläche des Dialysators ist eine Kupferfolie aufgebracht, die als Streifenleiter S1, S2 dient und die in Richtung der Längserstreckung des Dialysators 10 verläuft.

Die Anordnung von Streifenleiter in direkter Anbindung an den Dialysator oder an einer sonstige Blutreinigungsvorrichtung bringt den Vorteil mit sich, dass sich innerhalb der Blutreinigungsvorrichtung eine hohe Feldstärke erzielen lässt und dementsprechend eine besonders wirksame Abtrennung der urämischen Toxine von ihren Bindungspartner, insbesondere von Proteinen erfolgen kann. Dies wiederum ermöglicht die effiziente Abtrennung der Toxine aus dem Blut beispielsweise mittels eines Dialysators.

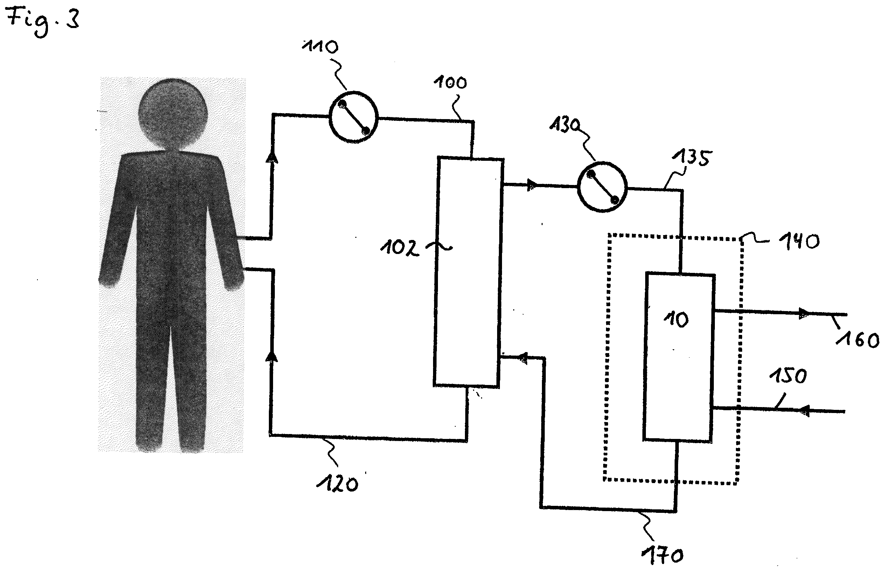

Figur 3 zeigt eine denkbare Ausführung einer Anordnung zur Blutreinigung.

Das Bezugszeichen 100 kennzeichnet eine Leitung, mittels derer Blut von einem Patientenzugang zu dem Plasmafilter 102 geführt wird. Zur Förderung des Blutes dient die Pumpe 110. Das Retentat des Plasmafilters 102 sowie das in dem Dialysator 10 gereinigte Blut wird über die Leitung 120 dem Patienten wieder zugeführt.

In dem Plasmafilter 102 erfolgt eine Trennung des Blutes in zelluläre Bestandteile und in Plasma, wobei die zellulären Bestandteile zurückgehalten werden und das Plasma die Filtermembran durchdringt.

Das in dem Plasmafilter 102 gewonnene Blutplasma wird mittels der Pumpe 130 über die Leitung 135 dem Dialysator 10 zugeführt.

Das Bezugszeichen 140 kennzeichnet symbolisch die auf dem Dialysator 10 angeordneten Streifenleiter S1, S2, die innerhalb des Dialysators 10 ein Feld und vorzugsweise ein hochfrequentes elektromagnetisches Feld erzeugen.

Wie oben ausgeführt, bewirkt das Feld eine Abtrennung der urämischen Toxine von Proteinen, insbesondere von Albumin, so dass der Anteil an freien urämischen Toxinen entsprechend gesteigert werden kann. Diese können über die Membran des Dialysators 10 abgeführt und von einer Dialyselösung aufgenommen werden, die die Hohlfasern des Dialysators 10 umströmt und die mittels der Leitungen 150, 160 zu bzw. von dem Dialysator hin- bzw. abgeführt wird.

Das in dem Dialysator 10 gereinigte Blut wird über die Leitung 170 zu dem Plasmafilter 102 zugeführt und dort mit den zellulären Bestandteilen, die von der Filtermembran des Plasmafilters 102 zurückgehalten wurden, gemischt und dem Patienten über die Leitung 120 wieder zugeführt wird.

Die Anordnung eines Plasmafilters vor dem Dialysator oder einer sonstigen Blutreinigungsvorrichtung bringt den Vorteil mit sich, dass die zellulären Bestandteile nicht dem elektromagnetischen Feld im Dialysator ausgesetzt werden, wodurch die Biokompatibilität des Verfahrens erhöht wird.

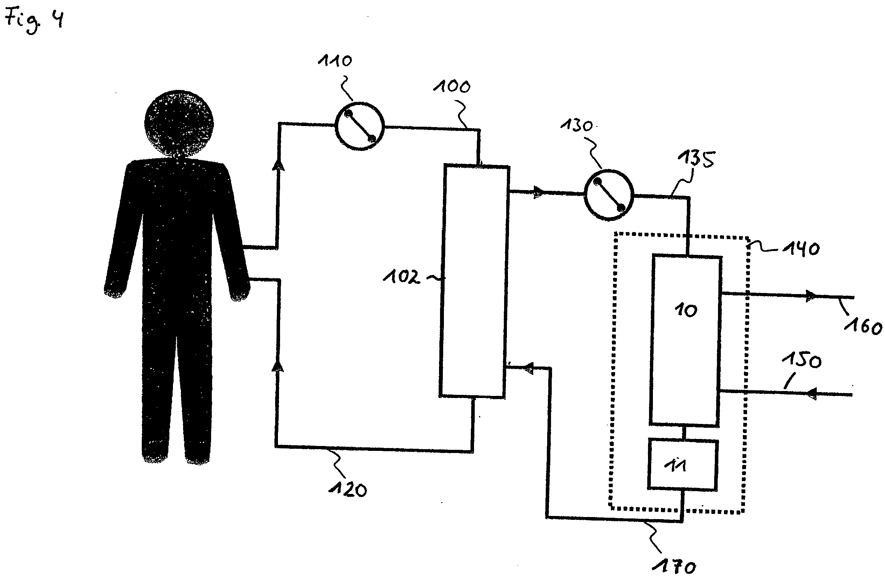

Figur 4 zeigt eine weitere Ausgestaltung einer Anordnung zur Blutreinigung, wobei gleiche Bezugszeichen wie in Figur 3 identische oder funktionsgleiche Bestandteile zeigen.

Wie dies aus Figur 4 hervorgeht, ist als Blutreinigungsvorrichtung nicht nur ein Dialysator 10, sondern zusätzlich ein Adsorber 11 vorgesehen, der vor oder nach dem Dialysator 10 angeordnet sein kann. Der Adsorber kann ebenfalls mit Streifenleitern versehen sein, so dass auch in dem Adsorber ein Feld vorliegt.

Der Adsorber hat die Aufgabe, im Feld freigesetzte Toxine, die nicht über die Dialysatormembran abgetrennt werden, zu adsorbieren und somit aus dem Blutkreislauf zu entfernen.

Die Streifenleiter sind in dem in Figur 4 dargestellten Ausführungsbeispiel sowohl an dem Dialysator 10 als auch den Adsorber 11 angeordnet.

Von der Erfindung ist auch eine Ausführungsform umfasst, bei der z.B. nur der Dialysator oder nur Adsorber mit Streifenleitern versehen ist.

Denkbar ist beispielsweise eine Ausführung, bei der der mit Streifenleitern versehene Adsorber dem Dialysator vorgeschaltet ist. In dem Adsorber findet in diesem Fall eine Freisetzung von Toxinen statt, die in dem Dialysator abgetrennt werden können.

Das Ausführungsbeispiel bezieht sich auf die Anordnung der Streifenleitern an einem Dialysator. Von der Erfindung sowie von dem Ausführungsbeispiel ist jedoch auch die Anordnung der Streifenleitern an beliebigen anderen Blutreinigungsvorrichtungen sowie an anderen Komponenten, insbesondere Leitungen umfasst. Diese befinden sich vorzugsweise in einem extrakorporalen Kreislauf, in dem das Blut eines Patienten oder ein Bestandteil, insbesondere das Blutplasma, einer Reinigung unterzogen wird.

## Patentansprüche

1. Vorrichtung zur extrakorporalen Entfernung proteingebundener Toxine aus dem Blut umfassend wenigstens eine Blutreinigungsvorrichtung, insbesondere wenigstens einen Dialysator, Hämofilter oder Adsorber, sowie wenigstens ein Mittel zur Erzeugung eines Feldes in der Blutreinigungsvorrichtung und/oder in einem mit der Blutreinigungsvorrichtung in Strömungsverbindung stehenden Element, insbesondere in einem mit der Blutreinigungsvorrichtung in Verbindung stehenden Leitungsabschnitt, **dadurch gekennzeichnet, dass** das Mittel wenigstens zwei Streifenleiter umfasst, die auf wenigstens zwei vorzugsweise gegenüberliegenden Aussenseiten der Blutreinigungsvorrichtung oder des Elementes angeordnet sind, so dass das Feld vorwiegend innerhalb der Blutreinigungsvorrichtung bzw. vorwiegend innerhalb des Elementes erzeugt wird.

2. Vorrichtung nach Anspruch 1, **dadurch gekennzeichnet, dass** die Streifenleiter derart mit der Blutreinigungsvorrichtung oder mit dem Element in Verbindung stehen, dass sich zwischen den Streifenleitern und der Blutreinigungsvorrichtung bzw. dem Element keine Luft befindet.

3. Vorrichtung nach Anspruch 1 oder 2, **dadurch gekennzeichnet, dass** die Streifenleiter mit der Blutreinigungsvorrichtung bzw. mit dem Element verklebt sind oder an der Blutreinigungsvorrichtung bzw. an dem Element mechanisch befestigt sind.

4. Vorrichtung nach einem der vorhergehenden Ansprüche, **dadurch gekennzeichnet, dass** sich zwischen den Streifenleitern und der Blutreinigungsvorrichtung bzw. dem Element ein Gel befindet.

5. Vorrichtung nach einem der vorhergehenden Ansprüche, **dadurch gekennzeichnet, dass** die Mittel zur Erzeugung eines Feldes derart ausgebildet sind, dass ein elektromagnetisches Feld erzeugt wird.

6. Vorrichtung nach einem der vorhergehenden Ansprüche, **dadurch gekennzeichnet, dass** die Mittel zur Erzeugung eines Feldes derart ausgebildet sind, dass ein elektromagnetisches Feld mit einer Frequenz im Bereich von 1 MHz bis 1 GHz und vorzugsweise mit einer Frequenz im Bereich von 50 MHz bis 200 MHz und besonders bevorzugt mit einer Frequenz im Bereich von 100 MHz bis 120 MHz erzeugt wird.

7. Vorrichtung nach einem der vorhergehenden Ansprüche, **dadurch gekennzeichnet, dass** die Mittel zur Erzeugung eines Feldes derart ausgebildet sind, dass ein elektromagnetisches Feld mit einer Frequenz im Bereich von 110 bis 111 MHz erzeugt wird.

8. Vorrichtung nach einem der vorhergehenden Ansprüche, **dadurch gekennzeichnet, dass** das Mittel zur Erzeugung eines Feldes derart ausgebildet ist, dass das Feld eine Feldstärke im Bereich von bis zu 4000 V/m, vorzugsweise im Bereich von 2000 V/m bis 4000 V/m und besonders bevorzugt im Bereich von 3000 V/m bis 3200 V/m aufweist.

9. Vorrichtung nach einem der vorhergehenden Ansprüche, **dadurch gekennzeichnet, dass** die Blutbehandlungsvorrichtung oder das Element wenigstens bereichsweise eine gekrümmte Oberfläche aufweist und dass die Streifenleiter ebenfalls gekrümmt ausgebildet sind, so dass sie der Kontur der Oberfläche der Blutbehandlungsvorrichtung bzw. des Elementes folgen.

10. Vorrichtung nach einem der vorhergehenden Ansprüche, **dadurch gekennzeichnet, dass** das Mittel teilweise oder vollständig als Folie oder als plattenförmiges Element ausgebildet ist.

11. Vorrichtung nach einem der vorhergehenden Ansprüche, **dadurch gekennzeichnet, dass** das Mittel sich über die gesamte Länge der Blutbehandlungsvorrichtung bzw. des Elementes oder einen Teil der gesamten Länge, vorzugsweise über mehr als die Hälfte der Länge der Blutbehandlungsvorrichtung bzw. des Elementes erstreckt.

12. Vorrichtung nach einem der vorhergehenden Ansprüche, **dadurch gekennzeichnet, dass** die Vorrichtung wenigstens einen extrakorporalen Kreislauf aufweist, in dem sich die Blutbehandlungsvorrichtung befindet.

13. Vorrichtung nach Anspruch 12 **dadurch gekennzeichnet, dass** der extrakorporale Kreislauf wenigstens einen Filter umfasst, der eine Permeatseite und eine Retentatseite umfasst, wobei die Blutbehandlungsvorrichtung bzw. das Element mit der Permeatseite in Strömungsverbindung steht.

14. Vorrichtung nach einem der vorhergehenden Ansprüche zur Verwendung in der Behandlung des akuten oder chronischen Nierenversagens und/oder zur Verwendung in der Behandlung des akuten oder chronischen Leberversagens.

15. Vorrichtung nach einem der vorhergehenden Ansprüche zur Verwendung in der Behandlung einer Sepsis oder Vergiftung.

## Claims

1. An apparatus for the extracorporeal removal of protein-bound toxins from blood comprising at least one blood purification apparatus, in particular at least one dialyser, hemofilter or adsorber, as well as at least one means for generating a field in the blood purification apparatus and/or in an element in flow communication with the blood purification apparatus, in particular in a line section connected to the blood purification apparatus, **characterized in that** the means comprises at least two strip conductors which are arranged on at least two preferably oppositely disposed outer sides of the blood purification apparatus or of the element such that the field is predominantly generated within the blood purification apparatus or predominantly within the element.

2. An apparatus in accordance with claim 1, **characterized in that** the strip conductors are connected to the blood purification apparatus or to the element such that no air is located between the strip conductors and the blood purification apparatus or the element.

3. An apparatus in accordance with claim 1 or claim 2, **characterized in that** the strip conductors are adhesively bonded to the blood purification apparatus or to the element or are mechanically fastened to the blood purification apparatus or to the element.

4. An apparatus in accordance with one of the preceding claims, **characterized in that** a gel is located between the strip conductors and the blood purification apparatus or the element.

5. An apparatus in accordance with one of the preceding claims, **characterized in that** the means for generating a field are configured such that an electromagnetic field is generated.

6. An apparatus in accordance with one of the preceding claims, **characterized in that** the means for generating a field are configured such that an electromagnetic field is generated having a frequency in the range from 1 MHz to 1 GHz, and preferably having a frequency in the range from 50 MHz to 200 MHz, and particularly preferably having a frequency in the range from 100 MHz to 120 MHz.

7. An apparatus in accordance with one of the preceding claims, **characterized in that** the means for generating a field are configured such that an electromagnetic field is generated having a frequency in the range from 110 to 111 MHz.

8. An apparatus in accordance with one of the preceding claims, **characterized in that** the means for generating a field is configured such that the field has a field strength in the range from up to 4000 V/m, preferably in the range from 2000 V/m to 4000 V/m, and particularly preferably in the range from 3000 V/m to 3200 V/m.

9. An apparatus in accordance with one of the preceding claims, **characterized in that** the blood treatment apparatus or the element has a curved surface at least regionally; and **in that** the strip conductors are likewise curved so that they follow the contour of the surface of the blood treatment apparatus or of the element.

10. An apparatus in accordance with one of the preceding claims, **characterized in that** the means is partly or completely configured as a film or as a plate-like element.

11. An apparatus in accordance with one of the preceding claims, **characterized in that** the means extends over the total length of the blood treatment apparatus or of the element or over a part of the total length, preferably over more than half the length of the blood treatment apparatus or of the element.

12. An apparatus in accordance with one of the preceding claims, **characterized in that** the apparatus has at least one extracorporeal circuit in which the blood treatment apparatus is located.

13. An apparatus in accordance with claim 12, **characterized in that** the extracorporeal circuit comprises at least one filter which comprises a permeate side and a retentate side, with the blood treatment apparatus or the element being in flow communication with the permeate side.

14. An apparatus in accordance with one of the preceding claims for use in the treatment of the acute or chronic renal failure and/or for use in the treatment of acute or chronic liver failure.

15. An apparatus in accordance with one of the preceding claims for use in the treatment of sepsis or toxification.

## Revendications

1. Dispositif d'élimination extracorporelle de toxines liées aux protéines hors du sang comprenant au moins un dispositif d'épuration du sang, en particulier au moins un dialyseur, hémofiltre ou adsorbant, ainsi qu'au moins un moyen destiné à générer un champ dans le dispositif d'épuration du sang et/ou dans un élément se trouvant en liaison d'écoulement avec le dispositif d'épuration du sang, en particulier dans une partie de conduit se trouvant en liaison avec le dispositif d'épuration du sang, **caractérisé en ce que** le moyen comprend au moins deux lignes microrubans, qui sont disposées sur au moins deux côtés extérieurs, de préférence opposés, du dispositif d'épuration du sang ou de l'élément, de sorte que le champ est généré principalement dans le dispositif d'épuration du sang ou principalement dans l'élément.

2. Dispositif selon la revendication 1, **caractérisé en ce que** les lignes microrubans sont en liaison avec le dispositif d'épuration du sang ou avec l'élément de telle manière qu'il n'y a pas d'air entre les lignes microrubans et le dispositif d'épuration du sang ou l'élément.

3. Dispositif selon la revendication 1 ou 2, **caractérisé en ce que** les lignes microrubans sont collées au dispositif d'épuration du sang ou à l'élément ou sont fixées mécaniquement sur le dispositif d'épuration du sang ou sur l'élément.

4. Dispositif selon l'une des revendications précédentes, **caractérisé en ce qu'**un gel se trouve entre les lignes microrubans et le dispositif d'épuration du sang ou l'élément.

5. Dispositif selon l'une des revendications précédentes, **caractérisé en ce que** les moyens destinés à générer un champ sont conçus de telle manière qu'un champ électromagnétique est généré.

6. Dispositif selon l'une des revendications précédentes, **caractérisé en ce que** les moyens destinés à générer un champ sont conçus de telle manière qu'un champ électromagnétique avec une fréquence comprise dans la plage de 1 MHz à 1 GHz et de préférence avec une fréquence comprise dans la plage de 50 MHz à 200 MHz et de manière particulièrement préférée avec une fréquence comprise dans la plage de 100 MHz à 120 MHz est généré.

7. Dispositif selon l'une des revendications précédentes, **caractérisé en ce que** les moyens destinés à générer un champ sont conçus de telle manière qu'un champ électromagnétique avec une fréquence comprise dans la plage de 110 à 111 MHz est généré.

8. Dispositif selon l'une des revendications précédentes, **caractérisé en ce que** le moyen destiné à générer un champ est conçu de telle manière que le champ présente une intensité du champ comprise dans la plage allant jusqu'à 4000 V/m, de préférence dans la plage de 2000 V/m à 4000 V/m et de manière particulièrement préférée dans la plage de 3000 V/m à 3200 V/m.

9. Dispositif selon l'une des revendications précédentes, **caractérisé en ce que** le dispositif de traitement du sang ou l'élément présente au moins dans certaines zones une surface incurvée et **en ce que** les microbandes sont également réalisées incurvées, de telle sorte qu'elles suivent le contour de la surface du dispositif de traitement du sang ou de l'élément.

10. Dispositif selon l'une des revendications précédentes, **caractérisé en ce que** le moyen est réalisé en partie ou entièrement sous la forme d'une feuille ou d'un élément en forme de plaque.

11. Dispositif selon l'une des revendications précédentes, **caractérisé en ce que** le moyen s'étend sur toute la longueur du dispositif de traitement du sang ou de l'élément ou une partie de la longueur totale, de préférence sur plus de la moitié de la longueur du dispositif de traitement du sang ou de l'élément.

12. Dispositif selon l'une des revendications précédentes, **caractérisé en ce que** le dispositif comporte au moins un circuit extracorporel, dans lequel se trouve le dispositif de traitement du sang.

13. Dispositif selon la revendication 12, **caractérisé en ce que** le circuit extracorporel comprend au moins un filtre, qui comprend un côté perméat et un côté rétentat, le dispositif de traitement du sang ou l'élément étant en liaison d'écoulement avec le côté perméat.

14. Dispositif selon l'une des revendications précédentes, destiné à être utilisé dans le traitement de l'insuffisance rénale aiguë ou chronique et/ou à être utilisé dans le traitement de l'insuffisance hépato-cellulaire aiguë ou chronique.

15. Dispositif selon l'une des revendications précédentes, destiné à être utilisé dans le traitement d'une septicémie ou d'une intoxication.
